# EUROPEAN PATENT APPLICATION

(11) **EP 4 471 427 A1**
(43) Date of publication of application: **04.12.2024**
(21) Application number: 23305861.9
(22) Date of filing: 31.05.2023
(51) Int. Cl.: G01N 33/68

(54) **BIOMARKER FOR SPONDYLOARTHITIS DIAGNOSTIC AND RESPONSE TO TREATMENT**

(71) Applicant: Centre Hospitalier Universitaire de Nice, 06000 Nice (FR); Université Côte d'Azur, 06100 Nice (FR)
(72) Inventor: SEITZ-POLSKI, Barbara, 06340 La Trinite (FR); ROUX, Christian, 06100 Nice (FR); BRGLEZ, Vesna, 06850 Briançonnet (FR); GRAÇA, Daisy, 06000 Nice (FR); CREMONI-GAUCI, Marion Claire, 06790 Aspremont (FR); DELANNOY, Eléonore, 83420 La Croix Valmer (FR)
(74) Representative: Lavoix

(57) **Abstract**

The present invention relates to methods, for determining whether a subject suffers from spondyloarthritis (Spa), for predicting the response to IL-17A inhibitor of a subject suffering from Spa, and for monitoring a treatment with an IL-17A inhibitor of a subject suffering from Spa, based on measuring the level of IL-17A protein expression in a sample of stimulated cells of the innate and adaptive immune system.

## Description

The present invention relates to methods, for determining whether a subject suffers from spondyloarthritis (Spa), for predicting the response to IL-17A inhibitor of a subject suffering from Spa, and for monitoring a treatment with an IL-17A inhibitor of a subject suffering from Spa, based on measuring the level of IL-17A protein expression in a sample of stimulated cells of the innate and adaptive immune system.

Spondyloarthritis (Spa, also called spondyloarthropathy) is a group of joints and spine inflammatory diseases with various clinical manifestations. There have been considerable advances in the diagnosis, classification, and understanding of the pathophysiology of SpA. This led to the successful introduction of new agents for clinical treatment. The advent of biological treatments has drastically changed the management of SpA. Despite this revolution, approximately 40% of the patients fail to respond to therapy, highlighting the importance of having a decision-making tool to guide the practitioner's therapeutic choice.

Biological disease-modifying antirheumatic drugs (bDMARDs) are a group of medications commonly used in people with rheumatoid arthritis or with Spa. The choice of bDMARD depends on a number of factors that vary depending on the physician, including the stage and severity of the person's condition, the balance between possible side effects and expected benefits, other medical conditions, and personal preference. Before treatment begins, the patient and clinician should discuss the benefits and risks of each type of therapy, including possible side effects and toxicities, dosing schedule, monitoring frequency, and expected results. There are several types of biologics, each of which targets a specific type of molecule involved in this process. For example, etanercept, adalimumab, infliximab, certolizumab pegol, and golimumab can be used as tumor necrosis factor (TNF) inhibitors. Ixekizumab and Secukinumab are also commonly used as IL-17A inhibitors.

When a patient does not respond to a bDMARD due to lack of efficacy or poor tolerability, evidence suggests that switching to another bDMARD can be a safe and effective treatment strategy. However, data for clinicians switching between different bDMARDs are limited.

To date, there are no validated biomarkers or head-to-head randomized controlled trials (RCTs) of biologics to guide the choice of biologic agents for spondylarthritis (SpA) and more particularly for Axial spondyloarthritis (axSpA), a subset of spondyloarthritis. Recommendation refers to bDMARDS, including TNF inhibitors (TNFi) and IL-17 inhibitors, for patients with high disease activity despite the use (or intolerance/contraindication) of at least two non-steroidal anti-inflammatory drugs (NSAIDs) in SpA and failure of a strategy including methotrexate for psoriatic arthritis. A tool to help the physician decide which bDMARDs to be preferred for usage is still required. Said tool has to be easy to perform, with reliable results, and preferably be non-invasive. Moreover, it has to be performed quickly, so as to treat the concerned patients in a rapidly manner.

### SUMMARY OF THE INVENTION

The inventors have demonstrated that determining cytokines level after stimulation of immune cells, in patients with active SpA requiring biological treatment, is a valuable tool to assist the physician in the choice of therapy. First, they demonstrated the feasibility of cytokine assays following immune cells stimulation, in active SpA which requires biological treatment. Second, they showed the predictive character of this expression on the therapeutic response three months after the implementation of an anti-IL-17A biological treatment.

Thus, the present invention relates to a method for predicting the response to IL-17A inhibitor of a subject suffering from Spa, comprising:
(i) stimulating cells of innate and adaptive immune system from a sample from said subject,
(ii) determining the level of IL-17A in said sample of stimulated cells, and
(iii) deducing from the results of step (ii) whether the subject is a good responder.

The present invention also relates to a method for the monitoring of a treatment with an IL-17A inhibitor of a subject suffering from Spa, comprising:
(i) stimulating cells of innate and adaptive immune system from a first sample obtained from said subject at a first point in time,
(ii) determining the level of IL-17A in said first sample of stimulated cells,
(iii) stimulating cells of innate and adaptive immune system from a second sample obtained from said subject, wherein said second sample is collected at a second point in time,
(iv) determining the level of IL-17A in said second sample of stimulated cells, and
(v) deducing from the results of steps (ii) and (iv) whether the subject is a good responder to the treatment.

The present invention also relates to a method for determining whether a subject suffers from spondyloarthritis (Spa), comprising:
(i) stimulating cells of innate and adaptive immune system from a sample from said subject,
(ii) determining the level of IL-17A in said sample of stimulated cells, and
(iii) deducing from the results of step (ii) whether the subject suffers from spondyloarthritis.

Preferably, the methods of the invention are *in vitro* or *ex vivo* methods.

The present invention also relates to a kit comprising:
(i) means for stimulating cells of innate and adaptive immune system, and
(ii) means for detecting IL-17A.

### DETAILED DESCRIPTION OF THE INVENTION

### Definitions

As used herein, the term « subject » or « patient » means a mammal, preferably a human. The subject of the methods of the invention may be of any age and gender.

As used herein the term "spondyloarthritis" or "Spa" refers to a family of inflammatory rheumatic diseases that have key features in common, including genetic, anatomical target, inflammation, pain and stiffness in the spine and pelvic joints, enthesitis. The most common type of SpA is axial spondyloarthritis (axSpA), a term that covers both ankylosing spondylitis (AS) and nonradiographic axial spondyloarthritis (nr-axSpA). Both mainly represented by inflammation in joints in the spine and pelvis. A less common type of SpA is peripheral spondyloarthritis (pSpA), where people have arthritis outside the spine, especially in the big joints in their arms or legs. Thus, in a preferred embodiment of the methods of the invention, Spa is Axial spondyloarthritis or peripheral spondyloarthritis.

As used herein "a subject suffering from Spa" is a subject diagnosed as suffering from Spa by any method well known by the skilled person and responding to ASAS criteria.

As used herein the term "sample" refers to a sample from the subject comprising cells of innate and adaptive immune system, typically said sample is a blood sample, i.e. a whole blood sample such as a heparinized whole blood sample.

By "stimulating cells", it is meant herein exposing cells to conditions or molecules that will activate at least one cellular pathway. In particular, in methods of the invention, stimulation of cells of innate and adaptive immune system is performed with a combination of agonists, more particularly a combination of a Toll Like Receptor (TLR) agonist and a T-cell receptors (TCR) agonist.

By "agonist", it is meant a chemical that activates a receptor to produce a biological response. Thus, a Toll Like Receptor (TLR) agonist activates a Toll like receptor, and a T-cell receptors agonist activates a Toll like receptor a T-cell receptor. Preferably, in the invention the combination of Toll Like Receptor (TLR) agonist and T-cell receptors (TCR) agonist comprises a TLR7-8 agonist and an anti-CD3 antibody.

In a particular embodiment the combination of a TLR7-8 agonist and an anti-CD3 antibody is fixed on lyophilized spheres (such as LyoSphereTM, Qiagen).

By "cells of innate immune system", it is meant cells playing a role in the immunity of a subject in a nonspecific manner. Cells of innate immune system includes macrophages, dendritic cells, histiocytes, natural killer cells, eosinophils, basophils, neutrophils and mast cells.

By "cells of adaptive immune system", it is meant cells playing a role in the immunity of a subject in a specific manner. Adaptive immunity creates immunological memory after an initial response to a specific pathogen, and leads to an enhanced response to future encounters with that pathogen. Cells of adaptive immune system includes lymphocytes B and lymphocytes T (also named T-cells).

In a preferred embodiment of the invention, cells of innate and adaptive immune system comprises natural killer cells and T-cells.

Preferably, stimulating cells of innate and adaptive immune system in the methods of the invention is performed with a combination of a Toll Like Receptor (TLR) agonist and a T-cell receptors (TCR) agonist, i.e. cells of innate and adaptive immune system are exposed to said combination in conditions and for a time sufficient to activate these cells. Preferably, this step is performed with the combination of a TLR7-8 agonist and an anti-CD3 antibody as TCR agonist. Preferably, said TLR7-8 agonist is R848 (also known as Resiquimod, CAS Number 144875-48-9). Preferably, said anti-CD3 antibody is the Muromonab-CD3 Teplizumab and/or Otelixizumab and/or OKT3.

Preferably, said TLR7-8 agonist and TCR agonist are fixed on lyophilized spheres (such as LyoSphereTM, Qiagen).

Preferably samples are incubated for at least 16h with a combination of a Toll Like Receptor (TLR) agonist and a T-cell receptors (TCR) agonist. This incubation is preferably performed at a temperature of at least 30°C, preferably at 37°C.

Preferably in the methods of the invention, after stimulating the cells of innate and adaptive immune system of the sample, said sample is centrifugated and the supernatant is used for determining the level of IL-17A protein expression. For example, the sample is centrifuged at 2,000-3,000 × g for 15 min and the supernatant is harvested. Supernatant can then be stored before determining the level of IL-17A protein expression. In a preferred embodiment supernatant is stored at -20°C.

IL-17A or IL-17 or Interleukin-17A is a proinflammatory cytokine (also referred as CTLA8 in rodents). It is expressed by cells of innate and adaptive immune system. Its role has been discovered in inflammation, autoimmune diseases and host defense. Its sequence has been determined and is well-known to the skilled in the art (see for example the human IL-17A sequence accession number Q16552 on UniProt as available on May 17 2023). Its targeting is studied for the treatment of several diseases. Notably an anti- IL-17A antibody (Secukinumab, Cosentyx^{®} or Ixekizumab, Taltz^{®}) have been approved for the treatment of psoriasis, of ankylosing spondylitis, or of nonradiographic axial spondyloarthritis.

As used herein, the term "determination of the level of IL-17A" includes determination of the level of IL-17A protein expression and determination of the level of IL-17 mRNA. It refers to determinate or quantify the expression or presence of IL-17A. Methods for determining or quantifying the expression of a protein are well known in the art. In a preferred embodiment of the methods of the invention, the level of IL-17A determined is level of IL-17A protein expression.

mRNA of IL-17A can be detected by any method know by the person skilled in art. Typically, the nucleic acid contained in the sample may be extracted according to standard methods, for example using lytic enzymes or chemical solutions or extracted by nucleic-acid-binding resins following the manufacturer's instructions. The extracted mRNA may be subjected to coupled reverse transcription and amplification, such as reverse transcription and amplification by polymerase chain reaction (RT-PCR), using specific oligonucleotide primers that enable amplification of a region in said genes. Quantitative or semi- quantitative RT-PCR can be used. Extracted mRNA may be reverse-transcribed and amplified, after which amplified sequences may be detected by hybridization with a suitable probe or by direct sequencing, or any other appropriate method known in the art. Other methods of amplification include ligase chain reaction (LCR), transcription-mediated amplification (TMA), strand displacement amplification (SDA) and nucleic acid sequence based amplification (NASBA). mRNA can also be identified by RNAseq or by single cell RNAseq allowing the identification of the cell expressing the mRNA.

In particular, in the context of the present invention, mRNA of IL-17A is detected by RNAseq, single cell RNAseq or hybridation *in situ.*

The determination of the level of IL-17A protein expression may be performed by any known method in the art. For example, the level of IL-17A protein expression may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, high performance liquid chromatography (HPLC), size exclusion chromatography, solid-phase affinity, etc.

In a particular embodiment, such methods comprise contacting the sample, or the supernatant of the sample with a binding partner capable of selectively interacting with IL-17A protein, such as an anti-IL-17A antibody.

The term "determination of the level of IL-17A in a sample" herein refers to direct determination of the level of IL-17A in said sample or determination of the level of IL-17A in a fraction of said sample, such as its supernatant.

"IL-17A inhibitor" includes all inhibitors of IL-17A known to the skilled in the art. In particular, IL-17A inhibitor includes anti-IL-17A antibodies. Anti-IL-17A antibodies include Secukinumab and Ixekizumab. Preferably, the IL-17A inhibitor according to the invention is a monoclonal antibody or an antigen-binding fragment thereof, typically a monoclonal antibody or an antigen-binding fragment comprising a heavy chain variable region comprising CDR1, CDR2 and CDR3 regions constituted of SEQ ID NO: 1, SEQ ID NO: 2 and SEQ ID NO:3 respectively, and a light chain variable region comprising CDR1, CDR2 and CDR3 regions constituted of SEQ ID NO: 4, SEQ ID NO: 5 and SEQ ID NO:6 respectively.

Typically, IL-17A inhibitor is an anti-IL-17A antibody comprising a VH of sequence SEQ ID NO: 7 and a VL of sequence SEQ ID NO: 8, or a heavy chain of sequence SEQ ID NO: 9 and a light chain of sequence SEQ ID NO: 10. In a preferred embodiment, the anti-IL-17A monoclonal antibody is Secukinumab (CAS number 875356-43-7).

An "antibody" may be a natural or conventional antibody in which two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site.

"Complementarity Determining Regions" or "CDRs" refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

As used herein, a sequence "at least 85% identical to a reference sequence" is a sequence having, on its entire length, 85%, or more, in particular 90%, 91%, 92%, 93%, 94%, 95%, 96%, 97%, 98%, 99%, 99.5%, 99.6%, 99.7%, 99.8%, 99.9% or 100% sequence identity with the entire length of the reference sequence.

A percentage of "sequence identity" may be determined by comparing the two sequences, optimally aligned over a comparison window, wherein the portion of the polypeptide sequence in the comparison window may comprise additions or deletions (i.e. gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, e.g. using the algorithm of Needleman and Wunsch (1970) J. Mol. Biol. 48: 443. The percentage of sequence identity can be readily determined for instance using the program Needle, with the BLOSUM62 matrix, and the following parameters gap-open=10, gap-extend=0.5.

In the context of the invention, CDR/FR definition in an immunoglobulin light or heavy chain is to be determined based on Kabat definitions.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system. (http://www.bioinf.org.uk/abs/#cdrdef)

As used herein, the term "antibody" denotes conventional antibodies and antigen-binding fragment thereof, as well as single domain antibodies and fragments thereof, in particular variable heavy chain of single domain antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit and bovine. Single domain antibodies may be naturally occurring single domain antibodies known as heavy chain antibody devoid of light chains. In particular, Camelidae species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by in vitro expression while achieving high yield. Furthermore, nanobodies are very stable, and resistant to the action of proteases. The properties and production of nanobodies have been reviewed by Harmsen and De Haard (2007) Appl. Microbiol. Biotechnol. 77:13-22.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, *i.e.* produced by protein engineering.

"Fragments" of antibodies comprise a portion of an intact antibody, in particular the antigen binding region or variable region of the intact antibody. Examples of antibody fragments include Fv, Fab, F(ab')₂, Fab', dsFv, (dsFv)₂, scFv, sc(Fv)₂ and diabodies, formed from antibody fragments. A fragment of a conventional antibody may also be a single domain antibody, such as a heavy chain antibody or VHH.

The term "Fab" denotes an antibody fragment having a molecular weight of about 50,000 Da and antigen binding activity, in which about a half of the N-terminal side of H chain and the entire L chain, among fragments obtained by treating IgG with a protease, papain, are bound together through a disulfide bond.

The term "F(ab')₂" refers to an antibody fragment having a molecular weight of about 100,000 Da and antigen binding activity, which is slightly larger than the Fab bound via a disulfide bond of the hinge region, among fragments obtained by treating IgG with a protease, pepsin.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂.
"dsFv" is a VH::VL heterodimer stabilized by a disulphide bond.
"(dsFv)₂" denotes two dsFv coupled by a peptide linker.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

The antibody preferably comprises a monoclonal antibody (mAb) or an antigen-binding fragment thereof, wherein the mAb or its fragment specifically binds to IL-17A. The mAb may be a murine, a chimeric, a humanized or a full-human monoclonal antibody. The fragment may be any type of mAb fragment that keeps substantially the ability of the whole antibody to bind to IL-17A, it can be for example a Fab or a F(ab')₂.

A "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass.

The term "humanized antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin. As used herein, the term "humanized antibody" refers to a chimeric antibody which contain minimal sequence derived from non-human immunoglobulin, e.g. the CDRs.

The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modeling, statistical analysis and mutagenesis to alter the non-CDR surfaces of antibody variable regions to resemble the surfaces of known antibodies of the target host.

Antibodies can be humanized using a variety of other techniques including CDR-grafting, veneering or resurfacing, and chain shuffling. Human antibodies can be made by a variety of methods known in the art including phage display methods.

The anti-IL-17A antibodies of the invention may be defined by their CDRs.

In the context of the invention, the term "treating" or "treatment" means reversing, alleviating, inhibiting the progress of the disorder or condition to which such term applies, or one or more symptoms of such disorder or condition.

As used herein the term "Th1 cytokines" denotes cytokines secreted by Th1 cells, it comprises IFN-y, IL-12p70, TNF- α, and IFN-α.

### A method for predicting the response to an IL-17A inhibitor of a subject suffering from Spa

The present invention relates to a method, preferably an *in vitro* or *ex vivo* method, for predicting the response to IL-17A inhibitor of a subject suffering from Spa, comprising:
(i) stimulating cells of innate and adaptive immune system from a sample from said subject,
(ii) determining the level of IL-17A in said sample of stimulated cells, and
(iii) deducing from the results of step (ii) whether the subject is a good responder.

Preferably, the subject is a subject suffering from Spa and in need of a treatment for treating Spa.

Preferably, the sample is obtained before the start of any treatment and in particular before the start of any treatment of Spa. Especially, the subject of the invention is suffering from Spa, and has not received any prior treatment.

In a preferred embodiment, the sample is obtained before the beginning of a treatment with an IL-17A inhibitor, typically before any treatment comprising a monoclonal antibody or an antigen-binding fragment comprising:
- a heavy chain variable region comprising CDR1, CDR2 and CDR3 regions constituted of SEQ ID NO: 1, SEQ ID NO: 2 and SEQ ID NO:3 respectively, and a light chain variable region comprising CDR1, CDR2 and CDR3 regions constituted of SEQ ID NO: 4, SEQ ID NO: 5 and SEQ ID NO:6 respectively, or
- a VH of sequence SEQ ID NO: 7 and a VL of sequence SEQ ID NO: 8, or
- a heavy chain of sequence SEQ ID NO: 9 and a light chain of sequence SEQ ID NO: 10;
   and more preferably before any treatment comprising Secukinumab (CAS number 875356-43-7)

Alternatively, the sample is obtained during treatment of the subject for Spa.

In a preferred embodiment, the level of IL-17A determined in step (ii) is level of IL-17A protein expression.

The method for predicting the response to IL-17A inhibitor of a subject suffering from Spa of the invention comprise a step of deducing from the results of step (ii) whether the subject is a good responder, said step is preferably performed by comparing the level of IL-17A with a predetermined reference value. Said comparison is indicative whether the subject is a good responder to an IL-17A inhibitor.

The predetermined reference value may be determined in a statistical analysis, as it is the case in the example. As used herein, the term "predetermined reference value" means a level of IL-17A which is determined for the method of the invention. In one particular embodiment, the reference value is determined by the addition of two standard deviation to the mean value of the IL-17A level (m+2o) of several samples of subjects, in particular subjects known for being poor responders. In another embodiment, the reference level is the optimal threshold value determined by ROC analysis. According to the invention, a reference level may be determined by a plurality of samples, preferably more than 5, 50, 100, 200 or 500 samples.

According to the method of the invention, the predetermined reference value for the level of IL-17A protein expression is preferably 73 pg ml⁻¹. Said predetermined reference value for the level of IL-17A protein expression may be slightly different from this value.

Preferably, the method for predicting the response to an IL-17A inhibitor of a subject suffering from Spa according to the invention comprises a step iii. of deducing from the results of step (ii) whether the subject is a good responder; said determination is made according to the level of IL-17A:
If the level of IL-17A is higher than its predetermined reference value, preferably if the level of IL-17A protein expression is higher than 73 pg ml⁻¹, then it is indicative that the subject is a good responder to a treatment with IL-17A inhibitor. By « good responder », it is meant a subject who will benefit from therapy with IL-17A inhibitors. In such a case, the use of at least one inhibitor of IL-17A, is the optimal first-line treatment. Preferably, the subject will be treated by an IL-17A inhibitor, and more preferably by an anti-IL-17A antibody, and in particular by Secukinumab.

If the level of IL-17A is equal or lower than its predetermined reference value, preferably if the level of IL-17A protein expression is lower than 73 pg ml⁻¹, then it is indicative that the subject is a poor responder to a treatment with IL-17A inhibitor. By « poor responder », it is meant a subject who will not benefit or will have too little benefit from therapy with IL-17A inhibitor. In such a case, the use of at least one inhibitor of TNF, is the optimal first-line treatment. Preferably, the subject will be treated by TNFi.

In other words, the present invention relates to an *in vitro* or *ex vivo* method for predicting the response to an IL-17A inhibitor, typically Secukinumab, of a subject suffering from Spa comprising:
(i) stimulating cells of innate and adaptive immune system from a sample from said subject, typically before any treatment with said IL-17A inhibitor
(ii) determining the level of IL-17A in said sample of stimulated cells,
(iii) comparing the level determined at step (ii) with a predetermined reference value and
iv) concluding that the subject is a good responder to said IL-17A inhibitor when the level determined at step (ii) is higher than the predetermined reference value; or concluding that the subject is a poor responder to said IL-17A inhibitor when the level determined at step (ii) is lower than the predetermined reference value.

### A method for the monitoring of a treatment with an IL-17A inhibitor

The present invention also relates to a method, preferably an *in vitro* or *ex vivo* method, for the monitoring of a treatment with an IL-17A inhibitor of a subject suffering from Spa, comprising:
(i) stimulating cells of innate and adaptive immune system from a first sample obtained from said subject at a first point in time,
(ii) determining the level of IL-17A in said first sample of stimulated cells,
(iii) stimulating cells of innate and adaptive immune system from a second sample obtained from said subject, wherein said second sample is collected at a second point in time,
(iv) determining the level of IL-17A in said second sample of stimulated cells, and
(v) deducing from the results of steps (ii) and (iv) whether the subject is a good responder to the treatment.

According to this method of the invention, the subject is a subject suffering from Spa. In an embodiment, the subject is a subject suffering from Spa and in need of a treatment for treating Spa.

In an embodiment, the subject starts its treatment of Spa between the collection of the two samples. In another embodiment, the subject has started a treatment Spa before the collection of the two samples.

In a preferred embodiment, the treatment of the subject includes an IL-17A inhibitor, preferably a monoclonal antibody or an antigen-binding fragment comprising:
- a heavy chain variable region comprising CDR1, CDR2 and CDR3 regions constituted of SEQ ID NO: 1, SEQ ID NO: 2 and SEQ ID NO:3 respectively, and a light chain variable region comprising CDR1, CDR2 and CDR3 regions constituted of SEQ ID NO: 4, SEQ ID NO: 5 and SEQ ID NO:6 respectively, or
- a VH of sequence SEQ ID NO: 7 and a VL of sequence SEQ ID NO: 8, or
- a heavy chain of sequence SEQ ID NO: 9 and a light chain of sequence SEQ ID NO: 10;
   and more preferably Secukinumab.

In the method according to the invention, the first sample is obtained before the second sample. Preferably, the second sample is obtained at least three weeks after the first sample, more preferably after 3 months.

In a preferred embodiment, the level of IL-17A determined in step (ii) and (iv) is the level of IL-17A protein expression.

The method for the monitoring of a treatment with an IL-17A inhibitor of a subject suffering from Spa according to the invention comprises a step v. of deducing from the results of steps (ii) and (iv) whether the subject is a good responder to the treatment; said determination is made according to the level of IL-17A:
If the level of IL-17A is equal or higher in the second sample than in the first sample, then it is indicative that the subject is a poor responder to the treatment. By « poor responder », it is meant a subject who does not benefit or have little benefit from the treatment. In such a case, the stop of the treatment with the IL-17A inhibitor might be considered.

If the level of IL-17A is lower in the second sample than in the first sample, then it is indicative that the subject is a good responder to the treatment. By « good responder », it is meant a subject who benefits from the therapy. Preferably, the subject will continue its treatment.

In other words, the invention relates to an *in vitro* or *ex vivo* method for monitoring the efficacy of a treatment with an IL-17A inhibitor of a subject suffering from Spa, comprising:
(i) stimulating cells of innate and adaptive immune system from a first sample obtained from said subject at a first time point,
(ii) determining the level of IL-17A in said first sample of stimulated cells,
(iii) stimulating cells of innate and adaptive immune system from a second sample obtained from said subject, at a second time point,
(iv) determining the level of IL-17A in said second sample of stimulated cells
(v) comparing the level of IL-17A determined in said first sample and the level of IL-17A determined in said second sample,
(vi) concluding when the level of IL-17A determined in said second sample is higher than the level of IL-17A determined in said first sample that the treatment is not efficient (i.e. the subject is a poor responder); or concluding when the level of IL-17A determined in said second sample is lower than the level of IL-17A determined in said first sample that the treatment is efficient (i.e. the subject is a good responder).

### A method for determining whether a subject suffers from spondyloarthritis (Spa)

Thus, the present invention relates to a method, preferably an *in vitro* or *ex vivo* method, for determining whether a subject suffers from spondyloarthritis (Spa), comprising:
(i) stimulating cells of innate and adaptive immune system from a sample from said subject,
(ii) determining the level of IL-17A in said sample of stimulated cells, and
(iii) deducing from the results of step (ii) whether the subject suffers from spondyloarthritis.

According to this method, the subject herein may present several symptoms, such as axial inflammatory manifestations, or peripheric inflammatory manifestations or enthesitic manifestations, but is not diagnosed has suffering from Spa.

In a preferred embodiment, the level of IL-17A determined in step (ii) is level of IL-17A protein expression.

The methods for determining whether a subject suffers from spondyloarthritis of the invention comprise a step (iii) of deducing from the results of step (ii) whether the subject suffers from spondyloarthritis, said step is preferably performed by comparing the level of protein expression with a respective predetermined reference value. Said comparison is indicative whether the subject suffers from spondyloarthritis.

As used herein, the term "predetermined reference value" means a level of IL-17A which is determined for the method of the invention. In one particular embodiment, the reference value is determined by the addition of two standard deviation to the mean value of the IL-17A level (m+2o) of several samples of subjects, preferably healthy donors. In another embodiment, the reference level is the optimal threshold value determined by ROC analysis. According to the invention, a reference level may be determined by a plurality of samples, preferably more than 5, 50, 100, 200 or 500 samples.

The method for determining whether a subject suffers from spondyloarthritis (Spa), according to the invention comprises a step iii. of deducing from the results of step (ii) whether the subject suffers from spondyloarthritis; said determination is made according to the level of IL-17A determined in step ii :
If the level of IL-17A is higher than its predetermined reference value, preferably if the level of IL-17A protein expression is higher than 162 pg.ml⁻¹, then it is indicative that the subject suffers from spondyloarthritis.

If the level of IL-17A is lower than its predetermined reference value, preferably if the level of IL-17A protein expression is lower than 162 pg.ml⁻¹, then it is indicative that the subject does not suffer from spondyloarthritis.

Said method may further comprise determining the level of at least one Th1 cytokine in said sample of stimulated cells. Indeed the inventors have shown that patient suffering from Spa have lower concentration of Th1 cytokines in stimulated samples.

### A kit

The present invention relates to a kit comprising:
(i) means for stimulating cells of innate and adaptive immune system, and
(ii) means for detecting IL-17A.

By " means for stimulating cells", it is meant herein means for activating at least one cellular pathway in cells. The means for stimulating cells of innate and adaptive immune system may for example comprise agonist and preferably a combination of agonists, more particularly a combination of a Toll Like Receptor (TLR) agonist and a T-cell receptors (TCR) agonist. Preferably, the means for stimulating cells according to the invention comprises the combination of Toll Like Receptor (TLR) agonist and T-cell receptors (TCR) agonist such as a TLR7-8 agonist and an anti-CD3 antibody. Preferably, said TLR7-8 agonist is R848 (also known as Resiquimod, CAS Number 144875-48-9). Preferably, said anti-CD3 antibody is the Muromonab-CD3 Teplizumab and/or Otelixizumab and/or OKT3.

By "means for detecting IL-17A" it is meant means that permit to determine the level of IL-17A protein or mRNA.

The determination of the level of IL-17A protein expression may be performed by any known method in the art. For example, the level of IL-17A protein expression may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, high performance liquid chromatography (HPLC), size exclusion chromatography, solid-phase affinity, etc.

In a particular embodiment, such means includes a binding partner capable of selectively interacting with IL-17A protein, such as an anti-IL-17A antibody.

In a particular embodiment, such means includes probes and primers that permit to determine the level of IL-17A mRNA.

The kit according to the invention may further comprise:
(iii) means for detecting a Th1 response, and/or
(iv) means to obtain a sample comprising cells of innate and adaptive immune system.

By "means for detecting a Th1 response", it is meant herein means that permit to determine the level of expression of at least one Th1 cytokine. The determination of the level of expression of cytokine may be performed by any known method in the art. For example, it may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, high performance liquid chromatography (HPLC), size exclusion chromatography, solid-phase affinity, etc.

In a particular embodiment, such means includes a binding partner capable of selectively interacting with said Th1 cytokines, such as an antibody specific to said Th1 cytokine.

By "means to obtain a sample comprising cells of innate and adaptive immune system", it is meant herein device use to obtain the sample from the subject or to store said sample such as a vial.

The present invention also concerns the use of a kit, preferably the *ex vivo* or *in vitro* use of a kit according to the invention, for predicting the response to IL-17A inhibitor, typically Secukinumab, of a subject suffering from Spa.

The present invention relates to the use of a kit according to the invention, preferably the *ex vivo* or *in vitro* use of a kit according to the invention, for monitoring of a treatment with an IL-17A inhibitor, typically Secukinumab, of a subject suffering from Spa.

The present invention relates to the use of a kit according to the invention, preferably the *ex vivo* or *in vitro* use of a kit according to the invention, for determining whether a subject suffers from Spa.

### Pharmaceutical composition and uses

Another object of the present invention is a pharmaceutical composition for use in the treatment of Spa in a subject, wherein said pharmaceutical composition comprises a treatment suitable for said subject, wherein said suitable treatment is selected by performing the method for determining whether a subject suffer from Spa is a good responder to a treatment with an IL-17A inhibitor, said method being as defined above, and selecting a treatment to which the subject will respond favorably.

The present invention also relates to a method for the treatment of Spa in a subject comprising:
- determining whether the subject is a good responder to a treatment with an IL-17A inhibitor, by performing the steps of the method for determining whether a subject is a good responder to a treatment with an IL-17A inhibitor, as defined above,
- selecting a treatment to which the subject will respond favorably, and
- administering to said subject said selected treatment.

Preferably, the subject is a subject suffering from Spa and in need of a treatment for treating Spa.

In particular, if the subject suffering from Spa is a good responder to a treatment with IL-17A inhibitor, a treatment comprising IL-17A inhibitor will be selected. Else if the subject suffering from Spa is not a good responder to a treatment with IL-17A inhibitor, then a treatment with without IL-17 inhibitor will be selected such as a treatment with TNF inhibitors.

The invention will be further illustrated by the following figures and examples. However, these examples and figures should not be interpreted in any way as limiting the scope of the present invention.

### FIGURES

**Figure 1****:** Baseline cytokine levels (TNF-α, IL-8, IL-6, and IL-1β) without stimulation in 45 patients with SpA compared using Mann Withney test to a cohort of 28 Healthy Donors (HD) paired by age. SpA: patients with spondyloarthritis
**Figure 2****:** Baseline cytokine profile of patients with SpA (n=45) compared using Mann Whitney test to healthy donor (n=96) after *ex vivo* non-specific stimulation paired by age. HD: Healthy donor; AS: patients with spondyloarthritis.
**Figure 3****:** Baseline IL-17A cytokine levels in IL-17-inhibitors responders compared using Mann Withney test to non-responders. Responders are defined three months after initiation of anti-IL-17A therapy by a BASDAI decrease in BASDAI of at least 50%.
**Figure 4****:** ROC curves evaluating the performance of measuring IL-17A produced after ex vivo non-specific stimulation to predict response to anti-IL-17A at 3 months after initiation of treatment (AUC: 0.80 p= 0.02)

### EXAMPLE 1:

### MATERIALS AND METHODS

### Study design and population

Prospective cohort study at the Rheumatology Department of the University Hospital of Nice.

All patients were recruited during rheumatology consultations between February 2021 and March 2022. The study was proposed to all patients with SpA meeting the assessment of spondylarthritis international society (ASAS) criteria who were to benefit from implementing a biological treatment after the failure of at least two NSAIDs. They had to have an active disease defined by a bath ankylosing spondylitis disease activity index (BASDAI) higher than 4, a predominant axial manifestation, no previous biological treatment, and no contraindication for the implementation of these treatments. After accepting participation in the study, all patients signed a non-opposition form. The study protocol complied with the ethical recommendations of the 1975 Declaration of Helsinki. The patients included in the study had a blood test and sociodemographic data collected (such as age, sex), as well as clinical data including the date of onset of the disease, the presence of human leukocyte antigen (HLA)-B27 or radiographic or magnetic resonance imaging-defined sacroiliitis; BASDAI; pain visual analog scale; c-reactive protein; presence of comorbidity, active smoking, axial, peripheral, or enthesitis manifestations; and extra-articular manifestations of the disease. The patients were then prescribed biological treatment with anti-TNF (adalimumab, golimumab, infliximab, etanercept, and certolizumab pegol) (TNFi group) or anti- IL-17 (Secukinumab) (IL-17i group). All patients were examined by the same investigator who decided on biotherapy based on their standards. The patients were then reviewed at one month to assess treatment tolerance and at three months to determine efficacy.

The control population was issued from a previews cohort of healthy subjects recruited between January 2020 and February 2021 from volunteer caregivers working at the Nice University Hospital. The controls were between 18 and 65 years of age and were free of inflammatory diseases. All the controls were interviewed using a questionnaire and blood tests.

### Blood Collection and Cytokine Assay

Cytokine assays were performed at the time of the pre-therapeutic assessment.

One milliliter of whole blood was collected and stimulated with immune ligands (anti-CD3 as a T-cell stimulant, R848 as TLR 7/8 agonist, or both) on single lyophilized spheres (LyoSphereTM, Qiagen) within 8 h of blood collection. Stimulated blood samples were incubated for 16-24 h at 37°C and then centrifuged at 2,000-3,000 × g for 15 min to harvest the stimulated serum. Stimulated serum was stored at -20°C until analysis, and freezethaw cycles were minimized to preserve the quality of the samples. Serum levels of 11 cytokines (IL-17A, IL-6, IL-1β, IFN-y, IL-12p70, TNF-α, IL-10, IL-5, IL-4, IL-13, and GM-CSF) were measured using the custom-designed cartridges Ella (ProteinSimpleTM), following the manufacturer's instructions. Ella measures cytokines in a microfluidic Simple Plex cartridge. All samples were measured as pure and diluted at 1:100, as the detection threshold differed among the cytokines. Blood was collected at baseline before any treatment and three months later.

### Assessments

The primary focus of these studies was on the feasibility of cytokine assays following lymphocyte stimulation in active SpA, requiring biological analysis to determine the correlation. Second, the correlation between the cytokine assay and the clinical response was studied. Therefore, efficacy endpoints were assessed through week 12. A significant clinical response was defined as an improvement of 50 or more of the initial BASDAI (BASDAI 50).

### Statistics

For descriptive statistics, data are presented as mean and standard deviation for quantitative variables with a Gaussian distribution, as median and range for quantitative variables with non-Gaussian distribution, or as numbers and percentages for qualitative variables. The Shapiro-Wilk test was used to determine whether a variable had a Gaussian distribution. Quantitative variables were compared using the unpaired t-test or one-way ANOVA if the values were normally distributed and the Mann-Whitney test if they were not. As appropriate, qualitative variables were compared using the chi-square test or Fisher's exact test. A receiver operating characteristic curve was used to define the IL-17A threshold above which patients were considered positive. The Wilcoxon matched-pairs signed rank test was used to compare two measurements of a quantitative variable performed on the same subjects. Statistical analyses were performed using GraphPad Prism 7.0 (GraphPad Software, Inc., San Diego, CA, USA) or SAS 9.0. Differences were considered significant when the p-value was < 0.05.

### RESULTS

Fifty-one patients with active SpA were recruited, and their cytokine profiles were analyzed during the pre-therapy workup.

Two assays could not be performed, and four patients did not relapse on the day of the assay.

A total of 45 patients with SpA (25 males and 20 females; median age, 41 ± 11 years) were included in the study, along with 27 controls (11 males and 16 females; median age 50.5 ± 28 years) for the analysis without *in vitro* stimulation and 96 healthy donors for the analysis with *in vitro* stimulation (median age 45.0 ± 12.5 years). The two populations were comparable in age (p = 0.43)).

Ultimately, three patients did not receive any biological treatment. Six patients were lost to follow-up (4 in the TNFi group and 1 in the IL-17i group) and could not be reassessed at three months. Finally, thirty-seven patients were reviewed three months after the initiation of biotherapy.

Twenty-eight patients were treated with TNF inhibitors (21 with adalimumab, 2 with etanercept, 2 with infliximab, 1 with golimumab, and 3 with certolizumab pegol), and 14 patients were treated with IL-17 inhibitor (Secukinumab). At three months, 55% of the treated patients had a BASDAI decrease of at least 50% (57.1% of the patients treated with TNFi and 64.3% treated with IL-17).

Patient characteristics at baseline and three months are detailed in **Table 1.**

**Table 1. Demographic, clinical, and paraclinical characteristics of patients with SpA.**

| **Characteristics** | **Total (n = 45)** | **TNFi (n = 28)** | **IL-17i (n = 14)** |
|---|---|---|---|
| **Age in years** - **median** ± **IQR** | 41 ± 11 | 41 ± 16 | 38 ± 15,2 |
| **Male gender** - **n (%)** | 25 (55,6%) | 10 (71,4%) | 7 (50%) |
| **HLA B27** ± **n (%)** | 19 (45,2%) | 12 (44,4%) | 5 (41,7%) |
| **CRP in mg/L** - **median** ± **IQR** | 5,0 ± 14,1 | 6,1 ± 20,1 | 4,4 ± 6 |
| **Sacroiliitis on radiography** - **n (%)** | 22 (48,9%) | 14 (50%) | 7 (50%) |
| **Sacroiliitis on MRI** - **n (%)** | 29 (64,4%) | 22 (91,7%) | 10 (100%) |
| **Cutaneous psoriasis** - **n (%)** | 13 (28,9%) | 7 (25%) | 6 (42,9%) |
| **History of uveitis** - **n (%)** | 5(11,1%) | 5 (17,9%) | 0 |
| **MICI** - **n (%)** | 3 (6,7%) | 2(7,1%) | 0 |
| **Active smoking** - **n (%)** | 14 (36,8%) | 11 (44%) | 2 (18,2%) |
| **Duration of disease in years** - **median** ± **IQR** | 3,0 ± 11 | 3,0 ±1 | 4,5 ± 8,5 |
| **Concomitant methotrexate treatment** - **n (%)** | 6 (13,3%) | 4 (14,3%) | 2 (18,2%) |
| **BADAI à M0** - **median ± IQR** | 6 ± 2 | 6,3 ± 2,4 | 6,4 ± 1 |
| **BASDAI à M3** - **median ± IQR** | 2,0 ± 3,7 | 2,1 ± 3,3 | 2,2 ± 3,9 |
| **Total number of responders at 3 months** - **n (%)** | 24 (64,8%) | 15 (62,5%) | 9 (69,2%) |

| | | | |
|---|---|---|---|
| IQR: interquartile range; n: number | | | |

### Cytokines levels in patients with SpA

### Serum cytokine levels in patients with SpA at baseline without stimulation of cells of innate and adaptive immune system

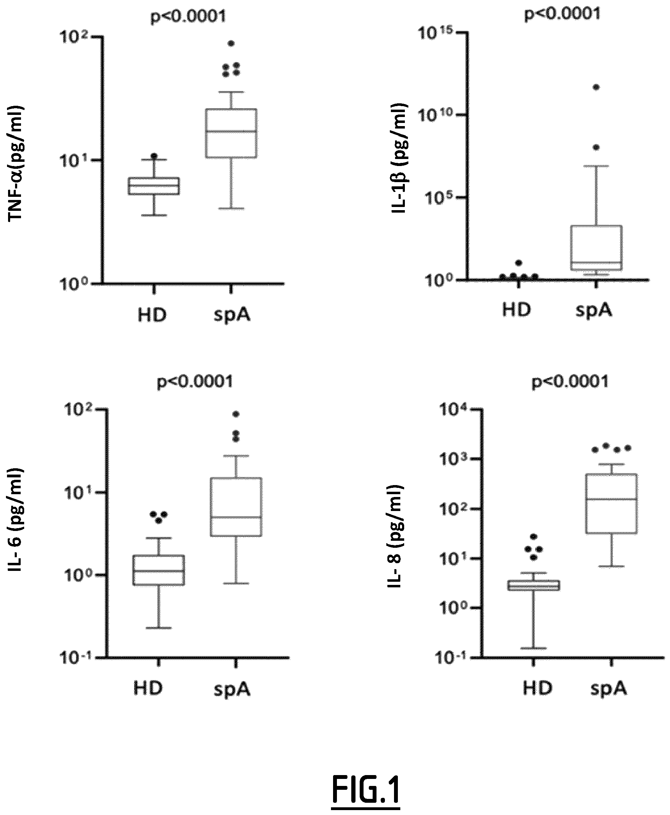

Levels of the pro-inflammatory cytokines TNF-α, IL-8, IL-6, and IL-1β were significantly higher in the patients with SpA group in comparison to controls (TNF-α 17,2 pg/mL [interquartile range [IQR], 17,2-26,9] versus 6,24 pg/mL [IQR 5,3-7,24], p < 0,001; IL-8 157,5 pg/mL [IQR 33,4-447,5] versus 2,73 IQR [2,27-3,61], p < 0,001; IL6 5 pg/mL IQR [3,1-13,2] versus 1,12 pg/mL IQR [0,75-1,7], p < 0,001; IL-1β 0,93 pg/mL IQR [0,5-3], versus 0,003 pg/mL IQR [0-0,06], p < 0,001) (Figure 1).

Without *in vitro* stimulation, IL-4, IL-5, IL-12p70, and IL-17A levels in all patients with SpA were below the detection threshold (<2,1 pg/mL for IL-17A).

### Cytokine levels in patients with SpA at baseline after in vitro stimulation of cells of innate and adaptive immune system

*In vitro* stimulation of TLR7/8 and T lymphocytes by anti-CD3 was induced to approximate the immune system reaction of patients with SpA and healthy donors, mimicking the activation of both innate and specific immune pathways.

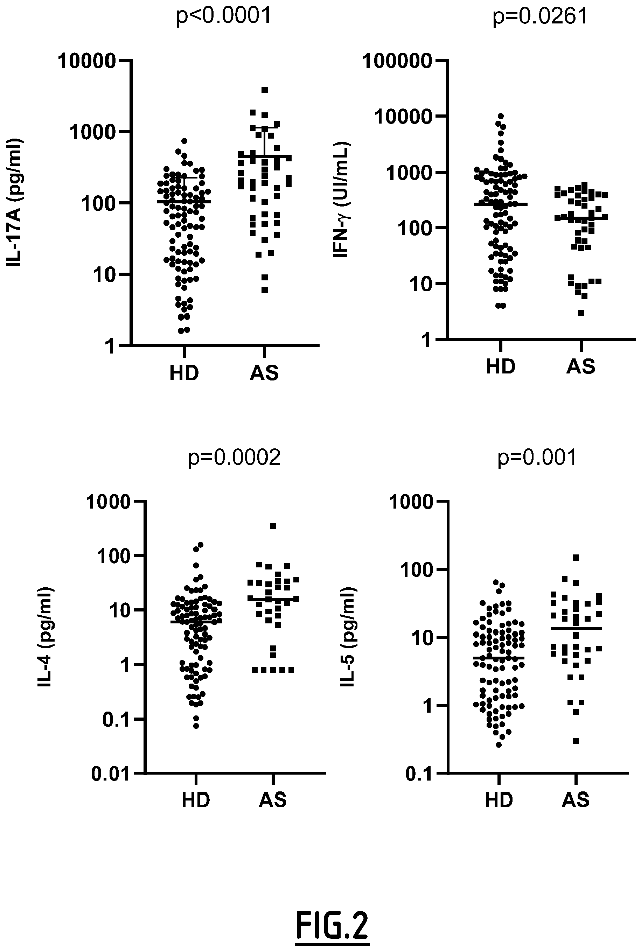

Supernatants concentration of IL-1β, IL-4, IL-5, TNF-α, IL12p70, INF-y and IL-17A were measured after *in vitro* stimulation (Figure 2).

Th17 cytokine IL-17A was significantly increased in the patient group with SpA in comparison to healthy donors (IL-17A 205 pg/mL IQR [63-467] versus 64 pg/mL IQR [15-146], p < 0,0001) (Figure 2).

Interestingly, the IL-17A levels of patients did not correlate with sex or age, the presence of extra-articular manifestations (psoriasis, IBD, or uveitis), the biological profile (HLA-B27, CRP), the radiological profile (sacroiliitis on X-ray or magnetic resonance imaging), duration of the disease, or exposure to methotrexate or smoking.

On the other hand, the concentration of Th1 cytokines, IFN-γ, IL-12p70 and TNF- α, and IL-1β, implicated in innate immune response, were lower in the patient group with SpA than in healthy donors (iFN-γ 149 UI/mL IQR [45-373] versus 268 pg/mL IQR [42-800], p = 0.0261) (Figure 2).

The cytokines of the Th2 pathway, IL-4, and IL-5, were higher in patients with SpA than in healthy donors (p =0.0002 and p = 0.001 respectively).

### Baseline IL-17A level after nonspecific in vitro stimulation, and three months response to IL-17i

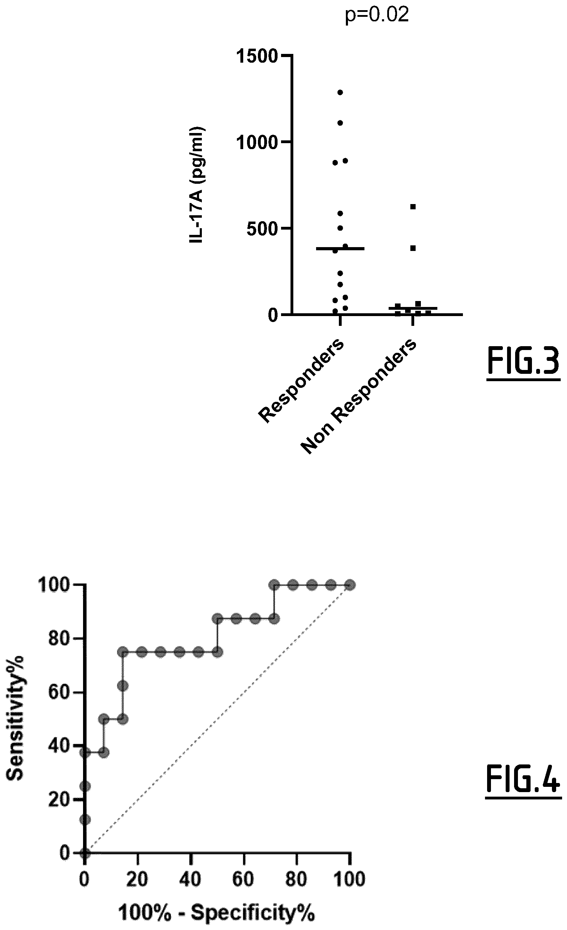

In patients with SpA, the baseline concentration of IL-17A was significantly higher in patients who responded to IL-17i treatment than in those who failed IL-17i treatment (382 pg/mL IQR [95-1288] vs. 37 pg/mL IQR [7-303] p = 0.02) (Figure 3).

A 73 pg/mL threshold was defined to distinguish the patients (responders and non-responders). Positive IL-17A activity was determined using receiver operating characteristic analysis, with a sensitivity of 75% and specificity of 88% (area under the receiver operating characteristic curve = 0,80 p = 0.02) (Figure 4). In our cohort, 31 out of 44 patients (70%) with IL-17A levels produced after non-specific *ex vivo* stimulation could have benefited from treatment with a specific IL-17A inhibitor, with a 3-month response rate of 85.7% in this group.

There was no difference in TNF-α levels, nor IL-17A levels, without or after stimulation between patients who responded to TNFi treatment and those who did not.

### Conclusion

This study shows the feasibility of a cytokine stimulation method that allows the determination of IL-17A levels in current practice in axial SpA and its interest in guiding the practitioner in their therapeutic choice (IL-17i or TNFi). The possibility of assaying plasma IL-17A requires an adapted methodology using non-specific stimulation of innate immune cells and T lymphocytes. As a matter of fact, without stimulation, the inventors found an elevation of TNF-α, d'IL1β, IL6, and IL8 in patients with SpA but not in IL-17A, which confirms the difficulty of measuring IL-17A. On the contrary, in patients with SpA, after stimulation, the IL-17A level was significantly higher than in controls, corresponding to strong activation of the Th17 pathway in these patients. The ability of the test to stimulate IL-17A via the stimulation of both acquired and innate immunity creates conditions like those encountered in diseases, including SpA. This explains the higher levels of IL-17 in this study and a discriminating difference in comparison with the levels found in the controls.

Furthermore, the inventors showed that the baseline IL-17A level was associated with a positive therapeutic response to anti-IL-17A inhibitor, in particular secukinumab, three months after the beginning of the treatment, as assessed by the BASDAI 50.

In conclusion, this study demonstrated the capacity of the assay to induce functional stimulation of IL-17A via innate and adaptive immune cells. This opens interesting perspectives in the management of SpA by providing a reliable assay of IL-17A, whose critical role for predictive the therapeutic response to anti- IL-17 inhibitors in SpA patient has been demonstrated in this study.

## Claims

1. An *in vitro* or *ex vivo* method for predicting the response to IL-17A inhibitor of a subject suffering from spondyloarthritis (Spa), comprising:
(i) stimulating cells of innate and adaptive immune system from a sample from said subject,
(ii) determining the level of IL-17A protein expression in said sample of stimulated cells, and
(iii) deducing from the results of step (ii) whether the subject is a good responder.

2. An *in vitro* or *ex vivo* method for the monitoring of a treatment with an IL-17A inhibitor of a subject suffering from Spa, comprising:
(i) stimulating cells of innate and adaptive immune system from a first sample obtained from said subject at a first point in time,
(ii) determining the level of IL-17A protein expression in said first sample of stimulated cells,
(iii) stimulating cells of innate and adaptive immune system from a second sample obtained from said subject, wherein said second sample is collected at a second point in time,
(iv) determining the level of IL-17A protein expression in said second sample of stimulated cells, and
(v) deducing from the results of steps (ii) and (iv) whether the subject is a good responder to the treatment.

3. The method according to claim 1 or 2, wherein the IL-17A inhibitor is an antibody, preferably the IL-17A inhibitor is a monoclonal antibody.

4. The method according to claim 3, wherein the IL-17A inhibitor is an anti-IL-17A monoclonal antibody, preferably wherein said antibody comprises a heavy chain variable region comprising CDR1, CDR2 and CDR3 regions constituted of SEQ ID NO: 1, SEQ ID NO: 2 and SEQ ID NO:3 respectively, and a light chain variable region comprising CDR1, CDR2 and CDR3 regions constituted of SEQ ID NO: 4, SEQ ID NO: 5 and SEQ ID NO:6 respectively.

5. The method according to claim 3 or 4, wherein the monoclonal antibody is secukinumab.

6. An *in vitro* or *ex vivo* method for determining whether a subject suffers from spondyloarthritis (Spa), comprising:
(i) stimulating cells of innate and adaptive immune system from a sample from said subject,
(ii) determining the level of IL-17A protein expression in said sample of stimulated cells, and
(iii) deducing from the results of step (ii) whether the subject suffers from spondyloarthritis.

7. The method according to claim 6, wherein said method further comprises determining the level of Th1 cytokines in said sample of stimulated cells.

8. The method according to any one of claims 1-7, wherein the sample is a whole blood sample.

9. The method according to any one of claims 1-8, wherein the subject is a mammal, preferably a human.

10. The method according to any one of claims 1-9, wherein the Spa is Axial spondyloarthritis or peripheral spondyloarthritis.

11. The method according to any one of claims 1-10, wherein stimulation of cells of innate and adaptive immune system is performed with a combination of a Toll Like Receptor (TLR) agonist and a T-cell receptors (TCR) agonist.

12. The method according to claim 11, wherein the combination of Toll Like Receptor (TLR) agonist and T-cell receptors (TCR) agonist comprises a TLR7-8 agonist and an anti-CD3 antibody.

13. A kit comprising:
(i) means for stimulating cells of innate and adaptive immune system, and
(ii) means for detecting IL-17A.

14. The kit according to claim 13, wherein means for detecting IL-17A comprises an anti-IL-17A antibody.

15. The method according to any one of claims 1-12, or the kit according to claim 13 or 14, wherein cells of innate and adaptive immune system comprises natural killer cells and T-cells.
